# EUROPEAN PATENT APPLICATION

(11) **EP 2 146 238 A1**
(43) Date of publication of application: **20.01.2010**
(21) Application number: 09446504.4
(22) Date of filing: 26.06.2009
(51) Int. Cl.: G02C 7/10, G02C 7/16

(54) **Device with glare shield**

(30) Priority: 11.07.2008 SE 0801672
(71) Applicant: Borgersen, Kjell, 471 60 Myggenäs (SE)
(72) Inventor: Borgersen, Kjell, 471 60 Myggenäs (SE)
(74) Representative: Andréasson, Ivar

(57) **Abstract**

The invention relates to a device 1 equipped with a glare shield for a user of the device. The device 1 is designed to be worn on a user's head, and comprises a holder 2 in which at least one transparent sheet 3 is mounted, for instance a lens of a pair of spectacles or a visor of a motorbike helmet. Through the sheet the user may view his environment when he wears the device on his head. The sheet 3 has two dark regions 4 blocking incoming light. The dark regions 4 are surrounded at least partly by the remaining part of the sheet 3 and are placed so that they coinside to a dark spot for the user's eye, which spot may dim a dazzling source of light. The invention relates also to a device comprising a pair of light-impermeable parts which may be clipped onto a pair of spectacles.

## Description

### TECHNICAL FIELD

The present invention relates to a device intended to be carried on a person's head and comprises one or several sheets, through which sheet or sheets a user of the device may view his environment. The device is designed to protect the user from being dazzled.

### BACKGROUND OF THE INVENTION

For instance, at car-driving the driver may be disturbed by the sun if the sun stays low in the sky. A known manner to counteract this is to place a hinged sunshield inside the roof of the car. However, such a hinged sunshield reduces the driver's field of view. Another known solution aims at the driver using sunglasses, i.e. spectacles with dark lenses taking away part of the sunshine. With sunglasses the field of view is not reduced but all light is blocked for the user and when the user of the sunglasses views something which is not directly illuminated this area is not seen so well. It has also been suggested in US 2007/0126255 that you may counteract dazzling of a driver in a vehicle by detecting the position of the driver's pupils in relation to the sun and then by moving two light blocking parts so that the sun cannot shine directly onto the driver's pupils. Such a device is, however, complicated and also bound to the vehicle. It is therefore an object of the present invention to provide an improved glare shield.

### DISCLOSURE OF THE INVENTION

The invention relates to a device with a glare shield which is designed to be worn on a person's head (a user's head). The device of the invention comprises a holder, in which holder at least one transparent sheet is mounted. Through the sheet (or sheets) a user of the device may view his environment when the user wears the device on his head. In the device of the invention the sheet (or sheets) has two regions with lower permeability to light than the remainder of the sheet/s. In this connection the expression "lower permeability to light" is to be interpreted such that these regions entirely or partly block incoming light. Each region with lower permeability to light is at least partly surrounded by the remaining part of the sheet, preferably at at least three side of the region with lower permeability to light. It shall be realized that the material of the surrounding part of the sheet at least partly allows incoming light to pass through. In many embodiments the material of the surrounding part of the sheet (or sheets) has a high permeability to light and admits all or almost all incoming light to pass through. The regions with lower permeability to light are placed so that they coincide to a dark spot for the user's eye. This dark spot may dim a source of light, e.g. the sun or a welding flame, which would otherwise dazzle the user. If the regions with lower permeability to light are designed entirely to block incoming light, they will coincide to a totally blind spot for the user's eye.

In a first embodiment the device may be a pair of spectacles. The holder is then the spectacle frame and the device then has two sheets which consist of the lenses of the spectacles, alternatively the device may consist of a pair of spectacles with only one sheet comprising two fields of view. The two regions with lower permeability to light are in this case situated on one sheet (lens) each or on one field of view each, if the spectacles have only one sheet.

Said regions with reduced permeability to light may be entirely impermeable to light, i.e. they may entirely block incoming light. This is normally the case when the invention is used on a pair of glasses even if spectacles are in principle conceivable where said regions with reduced permeability only block part of the incoming light.

In another embodiment the device may be a helmet, e.g. a motorbike helmet. The sheet may then be a visor of the helmet or the motorbike helmet. Also when the invention is used on a helmet, e.g: a motorbike helmet, said regions with reduced permeability to light are normally quite impermeable to light even if in principle a helmet is conceivable where said regions with reduced permeability only block part of the incoming light.

In a third embodiment the device may be a welding helmet. The regions with lower light-permeability may then have a certain permeability to light, suitably a permeability to light which is high enough for a user to be able to view the welding flame through the regions with lower permeability to light.

The two regions with lower permeability to light may be situated at a distance of 62 mm to 66 mm from each other.

When used on spectacles and helmets (e.g. motorbike helmets), it applies that the regions with lower permeability to light may have a width and height, respectively, of for instance 10 mm to 25 mm, more preferred a width of 12 mm to 22 mm, and a height of e.g. 10 mm to 22 mm. It shall be realized that the exact width and height may vary depending on the design and size of the device in other respects (e.g. design and size of the spectacle frame).

When applied to a welding helmet, the regions with lower light-permeability may suitably be somewhat larger, a suitable size may be 25 mm x 25 mm, for instance.

The invention may also have the form of a device designed to be attached to a pair of spectacles or on a visor of a motorbike helmet. Such a device comprises two parts being impermeable to light, and the device is arranged to be clipped on the upper part of a pair of spectacles or a visor so that each light-impermeable part blocks part of a lens or a visor. When the device 8 is clipped on the spectacles or the visor, each light-impermeable part blocks a region having a width of 10 mm to 25 mm, preferably a width of 12 mm to 22 mm, and a height of 10 mm to 22 mm.

Possibly, such a device may comprise a connection part connecting the two light-impermeable parts, wherein the distance between each vertical central line of the two light-impermeable parts (9) is 62 to 66 mm.

### BRIEF DESCRIPTION OF THE FIGURES

- Fig. 1: is a perspective view of a first embodiment of the invention;
- Fig. 2: principally shows a device for the measurement of the pupil distance (PD);
- Fig. 3: principally shows the function of the device of the invention;
- Fig. 4: is a perspective view of a second embodiment, where the invention is used on a helmet with a visor;
- Fig. 5: is a front view of a third embodiment where the invention is used on a welding helmet;
- Fig. 6: is a front view of an additional embodiment;
- Fig. 7: shows a side view of the embodiment of Fig. 6;
- Fig. 8: shows how the embodiment of Fig. 6 is used on a pair of spectacles;
- Fig. 9: shows a variant of the embodiment of Fig. 6.

### DETAILED DESCRIPTION OF THE INVENTION

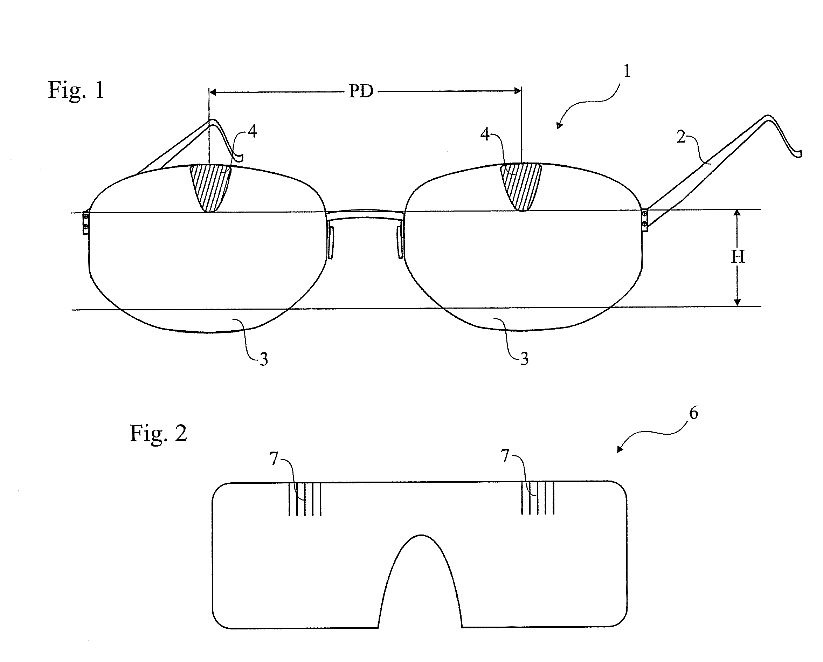

With reference to Fig. 1, the invention relates to a device 1 which is designed to be worn on a user's head. In the embodiment shown in Fig. 1 the device 1 is a pair of spectacles. The spectacles have a holder 2 in the form of a spectacle frame holding a pair of transparent sheets 3 being lenses, through which a user may view his environment when the user carries spectacles. Alternatively, the spectacles may have only one sheet with two fields of view. It should be realized that the expression "spectacle frame" in this context refers to each shape of physical structure fixing a pair of spectacle lenses so that they may be worn by a user , e.g. a pair of earpieces which are attached directly to the lenses. On each lens there is a dark region 4 with lower permeability to light than the remainder of the lens. For the spectacles it applies that the dark regions 4 normally are entirely impermeable to light. Each region 4 with lower permeability to light is surrounded at least partly by the remaining part of the sheet (lens) 3. It is suitable that the remaining part of the sheet 3 surrounds the region 4 with lower permeability to light on at least three sides. Fig. 1 shows how a region 4 with lower permeability to light on both its right and its left side, as well as its bottom side, is surrounded by the remaining part of the sheet 3 (the expressions "right", "left", and "bottom" relate here to what is "right", "left", and "bottom", respectively, in Fig. 1). As the region 4 with lower permeability to light on three sides is surrounded by the remainder of the sheet 3, the reduction of the field of view will be minimal, only the part of the field of view which contains a dazzling source of light is blocked while the remainder of the field of view is uninfluenced. The regions 4 with lower permeability to light are placed such that they coincide for the user's eye, or may be brought to coincide, to a entirely dark or entirely blind spot. The regions 4 with lower permeability to light are suitably placed above the normal field of view which designated H in Fig. 1.

As mentioned above, it applies at use on spectacles that the two regions 4 with lower permeability to light are entirely impermeable to light so that all incoming light is blocked. Embodiments of the invention are also conceivable where said regions 4 with lower permeability to light may be made of dark glass blocking only part of the light. Especially, this may be the case when the principle of the invention is applied to a welding helmet but also when the invention is applied to a pair of spectacles or to a helmet such as a motorbike helmet.

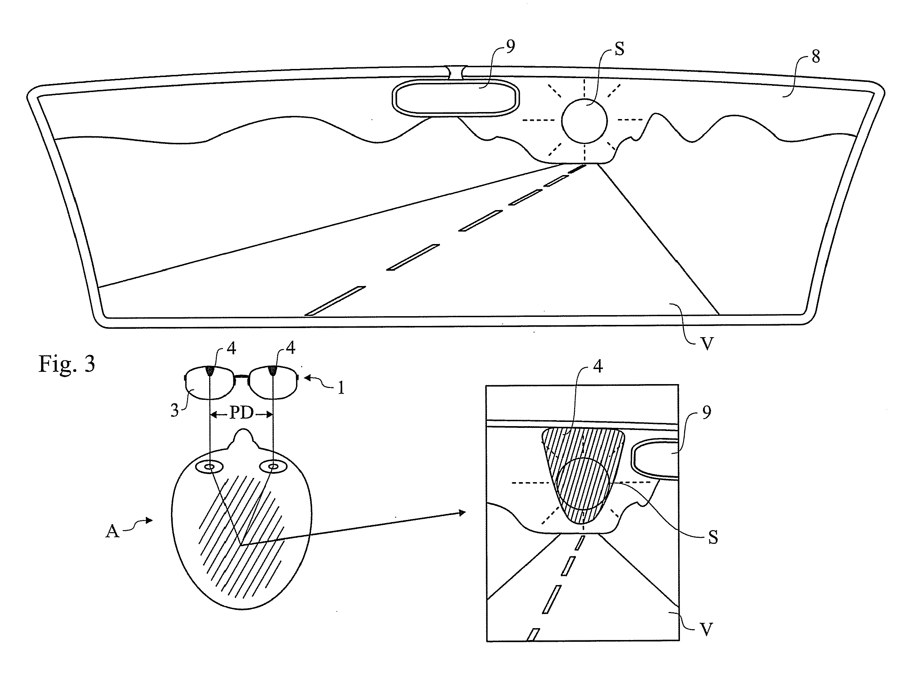

The function of the device of the invention will now be explained with reference to Fig. 3. In Fig. 3 it is schematically shown how a user A sitting in the driving seat of a car (not shown) views the road V through the windscreen 8 of the car. The sun S stays low and threatens to dazzle the driver of the car (the user A). The rear-view mirror 9 is not in such a position that it can block the sun. However, the driver wears a pair of spectacles designed according to the present invention. In the user's A field of view the two darker regions 4 will coincide so that they form a blind spot where the light from the sun is blocked. Only a blocked field (or light-reduced field in case that the regions 4 have a certain permeability) is seen in the middle of the upper edge of the field of view, as the human brain adds the two blocked fields to one field directed straight forward. If the sun does not shine directly towards the user's eyes but the sunshine will come somewhat obliquely from the front, the user may tum his head somewhat so that the dark regions 4 block the sun at the same time as the user turns his pupils forward towards the road. In this way, the user can avoid getting sunshine directly into his pupils at the same time as essentially the entire field of view outside the sun is visible without any obstacle. The blocking does not influence the normal function of the spectacles but may simply be used by the user bowing his head slightly forward, for instance for shading of the sunlight from the front at the same time as the user lifts his eye somewhat towards the road. As the user makes a small bow forward of his head, the darker parts 4 may come down into the normal field of view H and hence block a part thereof. When the user lifts its head again, the darker parts 4 will disappear from the field of view and become almost invisible for the user when he has been used to wear such spectacles.

The two regions 4 with lower permeability to light may suitably be situated at a distance of 62 mm to 66 mm from each other. For an average adult person the distance between the pupils is 64 mm and the distance between the two regions with lower permeability to light should correspond to the distance between the user's pupils. The normal variation of this distance is in the region of 62 mm to 66 mm for adult persons. The pupil distance (the PD-gauge) may be measured with an optic PD-meter for the determination of the distance between a person's pupils. Such a device is principally shown in Fig. 2. The measuring device shown in Fig. 2 may be designed to be placed on the nose of the person whose pupil distance is to be measured. In two regions 7 there is a measuring scale indicating distances. By means of this measuring scale you may determine the real distance PD (see Fig. 1) between the two regions 4 with lower permeability to light. It shall be realized that the distance PD between the two regions 4 with lower permeability to light do not relate to the smallest distance between these regions but to the distance between the centres of these regions, which shall be the same as the distance between the user's pupils (the pupil distance).

The regions 4 with lower permeability to light may have a width of 10 mm to 25 mm, for instance, preferably a width of 12 mm to 22 mm, and a height of 10 mm to 22, for instance. Here the width relates to the horizontal extension when the user wears spectacles and stands upright. The height here relates to the vertical extension when the user wears spectacles and stands upright. The sizes indicated (width 10 mm to 25 mm or 12 mm to 22 mm and height 10 mm to 22 mm) are such sizes which are suitable to block a source of light, as for example the sun, without hiding an unnecessarily large part of the normal field of view. The exact sizes may be determined from case to case depending on such factors as the design of the spectacle frames, the size of the lenses, and their distance from the pupils, or the physical size of the individual user. For instance, the regions 4 with lower permeability to light may have a conical shape which is indicated in Fig. 1 but also other shapes are, of course, conceivable.

A region with lower permeability to light may be achieved, for instance, by covering part of the lens with a dark tape or an adhesive sticker or by performing a coating of the lens, for instance by painting/spray-painting the lens. The lens may also be manufactured in such a way that part of the lens is made of dark glass forming a region 4 with lower permeability to light. Then, the spectacles are from the beginning manufactured for a certain user with a stipulated distance between the pupils.

A region 4, which is impermeable to light, may be achieved by providing an additional polarized film or sheet in the region of the lens of a pair of spectacles, which film/sheet is turned 90° in relation to the material of the remainder of the lens. Thereby all light is filtered away and a region 4 of the lens will become entirely impermeable to light. In a corresponding manner, said additional polarized film or sheet may be turned less than 90°, e.g. 45° to 85°, so that part of the light may pass.

As explained above, the regions 4 with lower permeability to light shall preferably on three sides be surrounded by the material of the remaining part of the sheet 3. Embodiments are very well conceivable where the regions 4 with lower permeability to light are entirely surrounded by the remainder of the sheet 3. However, it has proved that this may sometimes be inconvenient for the user. If the region with lower permeability to light is centrally located, this can in fact disturb the user's normal field of view. In preferred embodiments of the invention the regions with lower permeability to light therefore extend up to an upper edge of the sheet 3 (or the sheets 3). The regions with lower permeability to light are thus preferably arranged such that they on three sides are surrounded by the material of the remainder of the sheet (or sheets) and extend up to the upper edge of each sheet. They will then be located in the periphery of the sheet (or in the periphery of the respective sheet). In this way, the effect is achieved that the normal field of view is not influenced at all while the blocking/light-reducing function may be achieved by the user bending his head forwards. It is then enough for the user to make a small forward bend of his head.

The invention may be applied to spectacles cut to correct visual defects such as e.g. short-sightedness, astigmatism, or long-sightedness (or combinations of such visual defects) but may also be applied to pure sunglasses or to protective spectacles which are used in connection with sports or work.

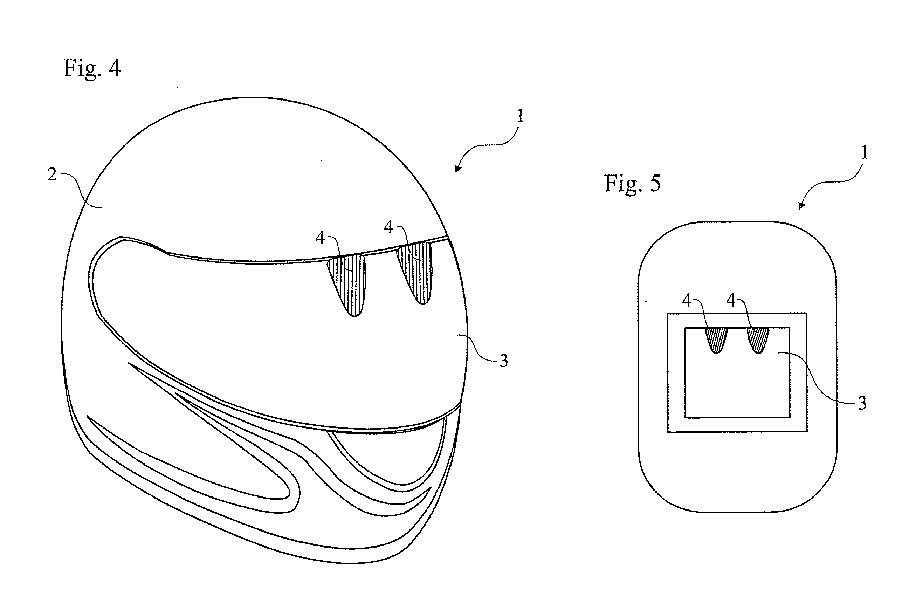

With reference to Fig. 4, it shall also be realized that the device of the invention may consist of a helmet, especially a motorbike helmet, wherein the sheet 3 consists of the visor of the motorbike helmet. The holder 4 consists in this case of the material of the helmet which protects the user's head and in which the visor is mounted. It is realized that this device protects against dazzling according to the same principle as explained above with reference to a pair of spectacles. It shall also be realized that the helmet must not necessarily be a motorbike helmet but also may be intended for other purposes. For instance, the helmet may be a helmet used by a cyclist or a car-driver. A pair of glare shields in the form of light-reducing or entirely light-blocking parts 4 may be arranged with hinges or be possible to push down on the upper part of visor. In an application on a helmet such as a motorbike helmet, the light-reducing/light-blocking regions 4 may suitably be somewhat larger than what they would be on a pair of spectacles, as the distance to the pupils is somewhat larger. It is realized that in an application to a helmet the invention will function in the same way as to a pair of spectacles. A user may be a motorcyclist, for instance, who in order to avoid being dazzled by the sun bends his head somewhat forward so that the dark regions 4 coincide to a blind spot hiding the sun while the remainder of the field of view remains uninfluenced.

As is indicated in Figs. 1 and 4, the dark or impermeable parts 4 may be conically narrowing downwards. In this way, the user may to a certain extent adjust the size of the dark or blind spot by bending his head differently far forward.

As is shown in Fig. 5, the device 1 may also be a welding helmet. The sheet 3 consists here of the window sheet of the welding helmet, in which there are two regions 4 with lower permeability to light which are partly surrounded by the remainder of the sheet 3. These regions may have certain permeability to light which is enough for the user to be able to view the welding flame through the region 4 or the regions 4 with lower permeability to light. Then, it is possible to have two fields of coloured protective glasses as protection against welding light. The other parts of the window of the welding helmet may be clear for inspection or made of a glass which is dark but not as dark as the two regions 4 with lower permeability to light. The user may view the welding flame during welding and the regions thereabout may be regarded without hindrance. When using such a welding helmet, the user may first view the region to be welded through a part of the (window) sheet 3, which has a relatively high permeability to light. When the user is prepared to begin the welding, he bends his head slightly forward so that the two regions 4 with lower permeability to light dim the region to be welded for the user's eye. Hence, the user's eyes are protected from the light from the welding flame. Therefore, the user may in an especially simple manner protect his eyes without being disturbed in his welding work. When applied to a welding helmet the two parts with lower permeability to light may be made of normal welding glass. Alternatively, the dark regions 4 may be made of such a material which automatically becomes darker when exposed to welding light. Such a material is marketed under the trade mark Speedglas™ and may be mentioned as an example of a possible material. At the application to a welding helmet, the regions 4 with lower permeability to light may suitably be somewhat larger than they normally are on a pair of spectacles or a motorbike helmet. On a welding helmet the regions 4 with lower permeability to light may possibly be square. They may then have the size 25 x 25 mm, for instance. On a welding helmet, the dark regions 4 preferably are square or rectangular rather than conically narrowing downwards, as this involves the advantage that a quicker dimming may thereby be achieved. However, other shapes than square are conceivable also for welding helmets.

At the application to a welding helmet the distance between the dark regions 4 should be somewhat smaller than what is the case for a pair of spectacles or such a helmet as a motorbike helmet. This depends on the focusing distance from eyes to weld joint (about 1 meter or less), and a relatively large distance between the eyes and the window sheet 3 (the welding glass). The distance between the dark regions 4 of the sheet 3 of the welding helmet may advantageously be about 4 mm less than the distance between the user's pupils (the PD-gauge).

At use of the welding helmet of the invention, the region around the dark regions 4 may be transparent and permit an inspection of the result and facilitate preparation. When the user is going to weld, he bends his head somewhat forward so that the region where the welding flame is to take effect is hidden or is made darker as the dark regions 4 coincide to a dark spot. After welding the user may lift his head somewhat and the welded region will then come into the user's field of view for inspection.

It shall also be realised that for all embodiments the regions 4 with lower permeability to light may be made of a material, the light-reducing ability of which depends on the amount of light the material is exposed to, for instance a material becoming more light-reducing the more light it is exposed to.

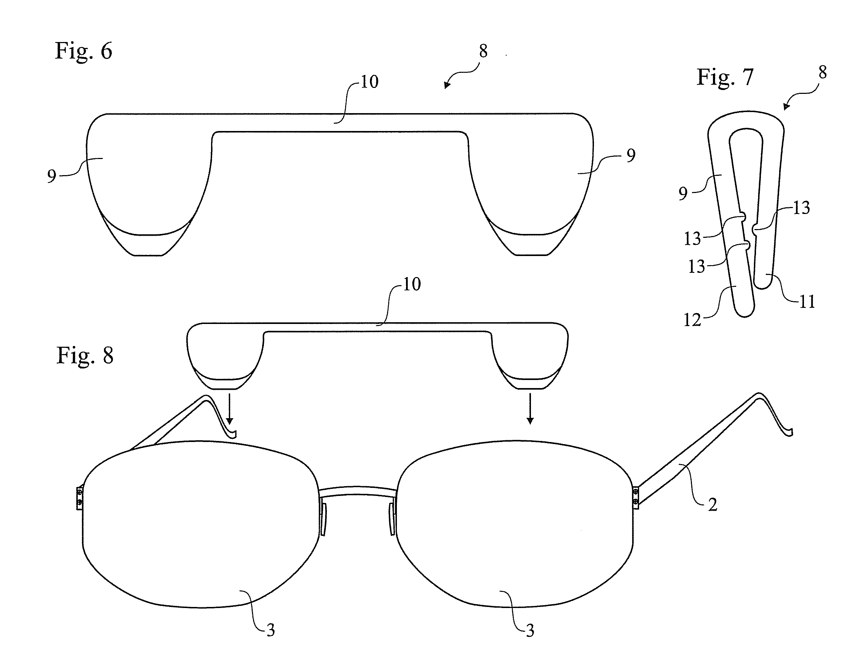

With reference to Fig. 6 to 8, an additional embodiment will now be explained. In Fig. 6 a device 8 is shown, which is designed to be attached to a pair of spectacles or on a visor of a motorbike helmet. The device 8 shown in Fig. 6 comprises two parts 9, which are impermeable to light, and said device is arranged to be clipped on the upper part of a pair of spectacles or a visor so that each light-impermeable part blocks part of a lens or a visor. When the device 8 is fixed to the spectacles or the visor, each light-impermeable part blocks a region having a width of 10 mm to 25 mm, preferably a width of 12 mm to 22 mm, and a height of 10 mm to 22 mm.

As is shown in Fig. 6, the device 8 may comprise a connection part 10 connecting the two light-impermeable parts 9. As is shown in Fig. 6, the two parts 9 which are impermeable to light are kept at a distance from each other by the connection part 10. This distance is suitably chosen in such a way that the distance between a vertical centre line of the two light-impermeable parts 9 is 62 mm to 66 mm. It is realized that the distance is then intended to correspond to the gauge PD, i.e. a user's pupil distance.

It is realized that when the device 8 is attached to a pair of spectacles or a visor, each one of the two parts 9 being impermeable to light will block a region of the visor, or a lens of the spectacles, which on three sides is surrounded by the material of the remainder of the visor or the lens and which extends up to an upper edge of the lens of the spectacles or the visor.

The device 8 may be designed so that the light-impermeable parts 9 are arranged to be clipped onto the lenses of a pair of spectacles (or to a visor). In Fig. 7 it is shown how a light-impermeable part 9 has two legs 11, 12 converging in a direction downwards against an open end which may be pressed down on a spectacle lens or a visor. The two legs 11, 12 may be resilient so that the legs 11, 12 are pressed towards each other when the part 9 is pressed down on a spectacle lens or a visor. For better attachment to the lens/visor the inside of the legs 11, 12 may have one or several protruding, small rims 13.

As indicated in Fig. 8, the device 8 may be pressed on the upper part of a pair of spectacles (or a visor). The function will then be the same as explained above with reference to Figs. 1 and 3. It is realized that the dark regions 4 according to Figs. 1, 3 or 4 in this case are achieved by the light-impermeable parts 9.

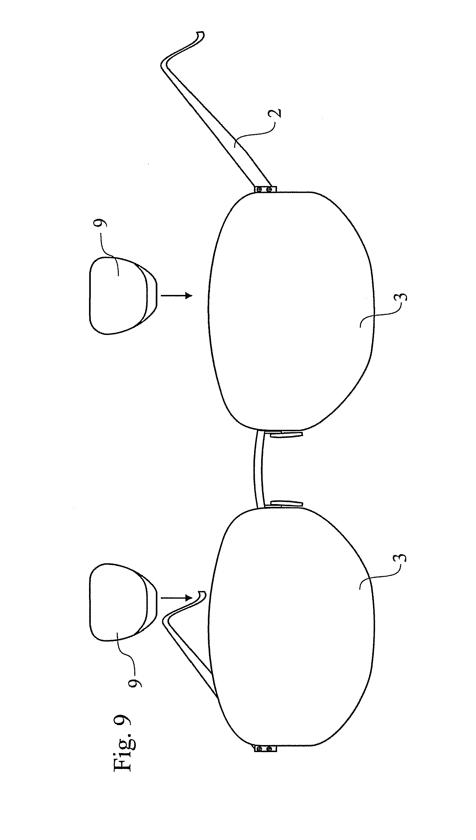

A variant of the embodiment shown in Figs. 6 to 8 is shown in Fig. 9. In the embodiment according to Fig. 9 there is no connection part 10 but there are two separate light-impermeable parts 9, each of which is clipped on a pair of spectacles or a visor to a motorbike helmet.

A device 8 according to Figs. 6, 7 or 9 may be provided for instance through extrusion of a rubber/plastic profile which is then formed by cutting. Also other methods, for instance casting, are conceivable.

With the invention a glare shield is obtained, which may be easily provided and at a low cost and which is not bound to a vehicle. The user's field of view is almost not at all influenced.

The invention is very advantageous at for instance car-driving or motorbike-driving in heavy sunshine when you suddenly drive into a dark or badly lit road tunnel. If you do not wear sunglasses, the pupils will contract so that you will see very badly in the moment driving into the tunnel. On the other side, if you wear sunglasses, they will become a hindrance inside the tunnel. With the present invention the user need only bend his/her head somewhat downwards when he/she is in the sunshine and then raise his head somewhat when he or she drives into the tunnel.

When the invention is used on a welding helmet you may among other things achieve the advantage that the size of the user's pupils need not change as much whether the user welds or not.

If the invention is realized in the form of a separate part which may be clipped on a pair of spectacles or a visor of a helmet, you may among other things get the advantage that you may move the glare shield from one pair of spectacles to another (or from one visor to another).

If you have such a connection part as shown in Fig. 6, i.a. the advantage is achieved that the light-impermeable parts 9 may be fixed at a correct distance from each other. However, this under the condition, that the device is adapted to a user with a certain pupil distance. With a connection part 10 according to Fig. 6 the advantage is also achieved that the device as a whole becomes easier to handle as compared to the variant according to Fig. 9; it is not so easy to lose separate parts.

If a device according to Fig. 9 is used, i.a. the advantage is obtained that the pupil distance may be adapted to any user.

## Claims

1. A device (1) designed to be worn on a user's head and which device (1) comprises a holder (2), in which holder (2) at least one transparent sheet (3) is mounted and through which sheet (3) a user of the device (1) may view his environment when the user wears the device on his head, **characterised in that** the sheet (3) has two regions (4) with lower permeability to light than the remainder of the sheet and which regions with lower permeability to light are at least partly surrounded by the remaining part of the sheet (3) and are placed so that they coincide to a dark or entirely blind spot for the user's eye.

2. A device (1) according to claim 1, **characterised in that** the device (1) is a pair of spectacles, wherein the holder (2) consists of the spectacle frame, and wherein the device (1) has two sheets (3) which are the lenses of the spectacles or one sheet (3) with two fields of view, and wherein the two regions (4) with lower permeability to light are located in one sheet (3) each or one field of view each.

3. A device (1) according to claim 1, **characterised in that** the regions (4) with lower permeability to light have a width of 10 mm to 25 mm, preferably 12 mm to 22 mm.

4. A device (1) according to claim 1, **characterised in that** the regions (4) with lower permeability to light have a height of 10 mm to 25 mm, preferably 10 mm to 22 mm.

5. A device according to claim 1, **characterised in that** said regions with lower permeability to light are entirely impermeable to light.

6. A device (1) according to claim 1, **characterised in that** the two regions (4) with lower permeability to light are located at a distance of 62 mm to 66 mm from each other.

7. A device (1) according to claim 1, **characterised in that** the device is a helmet and that the sheet (3) is a visor of the helmet.

8. A device (1) according to claim 1, **characterised in that** the device (1) is a welding helmet and that said regions (4) with lower permeability to light have necessarily high permeability to light so that a user will be able to view the welding flame through the regions (4) with lower permeability to light.

9. A device (8) designed to be clipped on a pair of spectacles or a visor of a motorbike helmet, which device (8) comprises two parts (9) being impermeable to light and which device (8) is arranged to be clipped onto the upper part of a pair of spectacles or onto a visor, so that each light-impermeable part 9, when the device 8 is fixed to the spectacles or the visor, blocks a region having a width and height, respectively, of 10 mm to 25 mm, preferably a width of 12 mm to 22 mm, and a height of 10 mm to 22 mm.

10. A device according to claim 9, **characterised in that** the device comprises a connection part (10) connecting the two light-impermeable parts (9) and that the distance between a vertical centre line of the two light-impermeable parts (9) is 62 mm to 66 mm.
